# EUROPÄISCHE PATENTANMELDUNG

(11) **EP 0 934 986 A1**
(43) Veröffentlichungstag der Anmeldung: **11.08.1999**
(21) Anmeldenummer: 97122248.4
(22) Anmeldetag: 17.12.1997
(51) Int. Cl.: C09B 69/00, C08B 37/00, C09B 69/10, G01N 33/58, G01N 33/52

(54) **Farbstoff-Polyaccharid- bzw. Cyclosaccharid-Konjugate und deren Verwendung als Diagnostikum**

(71) Anmelder: Roche Diagnostics GmbH, 68298 Mannheim (DE)
(72) Erfinder: Bosies, Elmar, Dr., 69469 Weinheim (DE); Hein, Heinz-Michael, Dr., 64342 Seeheim-Jugenheim (DE); Reiter, Rudolf, Dr., 69469Weinheim (DE)

(57) **Zusammenfassung**

Neue Farbstoff-Polysaccharid- bzw. -Cyclosaccharid-Konjugate und deren Verwendung als Diagnostikum, insbesondere zur Bestimmung der glomerulären Filtrationsrate beim Menschen.

## Beschreibung

Die vorliegende Erfindung betrifft neue Farbstoff-Polysaccharid- bzw. Cyclosaccharid-Konjugate sowie deren Verwendung als Diagnostikum, insbesondere zur Bestimmung der glomerulären Filtrationsrate beim Menschen.

Im Zusammenhang mit diagnostischen und therapeutischen Maßnahmen im präklinischen und klinischen Bereich kommt der Bestimmung der glomerulären Filtrationsrate eine große Bedeutung zu, weil sich mit ihrer Hilfe die Einschränkung der Nierenfunktion bestimmen läßt. Unter der glomerulären Filtrationsrate (GFR) versteht man die von den Glomerula der Nieren pro Zeiteinheit produzierte Menge Primärharn, die mit jedem Stoff ermittelt werden kann, der ausschließlich filtriert wird und weder tubulär sezerniert noch aus dem Primärharn rückresorbiert wird.

Zur Quantifizierung der glomerulären Filtrationsrate wird die Clearance der verwendeten Testsubstanz untersucht. Als Clearance bezeichnet man diejenige Plasmamenge in ml, die durch die Nieren in der Zeiteinheit (min) von einer bestimmten (Test-) Substanz befreit wird.

Es werden zur Zeit verschiedene Methoden angewendet, um diese Clearance zu bestimmen. So haben die endogene Kreatinin-Clearance, die Inulin-Clearance oder die ⁵¹Cr-EDTA (Na₂ ⁵¹Cr-ethylendiamintetraacetat)-Clearance Bedeutung erlangt. Auch Röntgenkontrastmittel wie Iohexol werden zur Bestimmung der Nierenclearance eingesetzt.

Alle aufgeführten Methoden haben jedoch gravierende Nachteile. So ist je nach Ausführungsart der jeweiligen Clearance-Bestimmung eine Dauerinfusion der Testsubstanz zur Aufrechterhaltung eines konstanten Plasmaspiegels und/oder ein mit unangenehmer Katheterisierung verbundenes Sammeln von Urin und/oder eine mehrmalige Blutentnahme nötig. Die Bestimmung der Inulinkonzentration im Plasma oder Urin ist ein aufwendiges Verfahren und mit einer relativ großen Fehlerbreite behaftet. Die Verwendung von radioaktiv markierten Substanzen stellt eine zusätzliche Belastung für den Organismus dar und sollte daher möglichst vermieden werden.

Als eleganteste Form der Clearance-Bestimmung wird die sogenannte Input-Clearance angesehen. Hierbei wird nicht die Ausscheidung eines Markers direkt gemessen, sondern man hält die Plasmakonzentration des Markers konstant. Die erforderliche Menge an Marker, die benötigt wird, um die Plasmakonzentration konstant zu halten, entspricht der über die Nieren ausgeschiedenen Markermenge und damit der Clearance. Hierbei muß naturgemäß die Plasmakonzentration kontinuierlich überwacht werden, was heutzutage nur mit Hilfe radioaktiv markierter Substanzen machbar ist.

Eine deutliche Verbesserung läge in der Verwendung von nicht radioaktiv markierten Markern, deren Plasmakonzentration kontinuierlich überwacht werden könnte. Hierdurch könnte auch auf die Blutentnahme bzw. das Urinsammeln zur Konzentrationsbestimmung des Markers verzichtet werden.

Die Aufgabe bestand daher darin, einen nicht radioaktiv markierten Marker zu finden, der vollständig durch renale Ultrafiltration eliminiert wird, also weder tubulär sezerniert noch rückresorbiert wird, und dessen Plasmakonzentration durch ein invasives oder nicht invasives Verfahren kontinuierlich bestimmt werden kann, wobei das nicht invasive Verfahren bevorzugt ist und die Bestimmung der Plasmakonzentration insbesondere durch transkutane Absorptionsmessungen vorgenommen wird.

Die vorliegende Erfindung löst dieses Problem durch Verwendung von Polysacchariden bzw. Cyclosacchariden, die kovalent mit einem Farbstoff verknüpft sind, wobei die Plasmakonzentration des Gesamtmoleküls mit Hilfe eines Detektors transkutan durch Absorptionsmessung bestimmt wird.

In der Literatur sind bereits einige derartige Verbindungen beschrieben. So gibt es Konjugate des Farbstoffes Cibacron Blau mit Amylose, Glucanen und Cellulose (Anal. Biochem. 31, 412 (1969); Acta Chem. Scand. 25, 298 (1971)); Biochem. J. 87, 90 (1963), das sogenannte Blue Dextran (Anal. Biochem. 39, 202 (1971) sowie den Farbstoff Remazole Brilliant Blue R in Verknüpfung mit Amylose (Experientia 23, 805 (1967)). In all diesen Farbstoff-Polysaccharid-Konjugaten handelt es sich bei dem Polysaccharid um ein aus Glucoseeinheiten aufgebautes Polysaccharid. Jedoch werden diese zum einen relativ schnell metabolisch abgebaut und eignen sich daher nicht für die Bestimmung der Nierenclearance. Zum anderen werden solche Polysaccharide nicht nur durch glomeruläre Filtration durch die Niere ausgeschieden und sind daher auch aus diesem Grunde für die Bestimmung der Nierenclearance nicht geeignet.

Als Polysaccharide im Sinne der vorliegenden Erfindung finden nun vor allem Polyfructosane wie z.B. Inulin, Levan, Asparagosin, Sinistrin, Fibrulin, Graminin, Phlein, Poan, Secalin und Irisin Verwendung, die durch glomuläre Filtration über die Niere ausgeschieden werden. Hierbei handelt es sich um Substanzen mit Kettenlängen von 10 - 30 Fructoseeinheiten, die teilweise am Ende einer Kette ein Glucosemolekül tragen. Vor allem werden Polysaccharide vom Typ des Inulins bzw. des besser wasserlöslichen Sinistrins eingesetzt.

Bei den verwendeten Cyclosacchariden handelt es sich um α-,β- oder γ-Cyclodextrin, vorzugsweise um das β-Cyclodextrin.

Bei der Farbstoffkomponente der Konjugate werden Farbstoffe eingesetzt, die eine funktionelle Gruppe wie z.B. eine Carboxyl-, Hydroxysulfonyl-, Amino-, Isothiocyanato- oder Isocyanatogruppierung tragen. Diese funktionellen Reste können direkt mit einem Polysaccharid zur Umsetzung gebracht werden oder über einen Spacer an das Polysaccharid gebunden werden.

Die im Sinne der Erfindung eingesetzten Farbstoffkomponenten der Konjugate haben ein Absorptionsmaximum im Bereich zwischen 500 und 1300 nm, vorzugsweise zwischen 600 und 900 nm. Hierfür eignen sich vor allem Anthrachhinon-, Phthalocyanin-, Phenazin-, Phenothiazin-, Phenoxazin-, Rhodamin-, Azo- und Cyaninfarbstoffe und deren Derivate. Repräsentative Vertreter einzelner Farbstoffe sind z.B. Cibacron Blau, Procion Blau MX-R, Cibacron Türkisblau GF-P und Indocyanin.

Gegenstand der Erfindung sind daher Verbindungen der allgemeinen Formel I 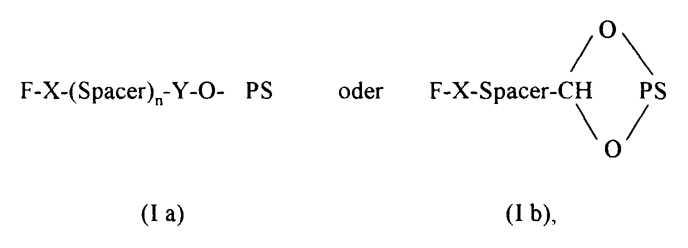 in der
F = eine Farbstoffkomponente mit einem Absorptionsmaximum zwischen 500 und 1300 nm, vorzugsweise zwischen 600 und 900 nm,
n = 0 oder 1,
X für den Fall, daß n = 0 ist, =
   -NH-C=O, -NH-C=S, -NH-C=NH, -CONH-C=S 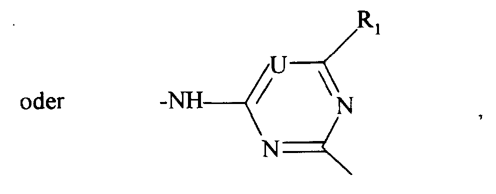
X für den Fall, daß n = 1 ist, =
   NH, -CONH, -SO₂NH, -NHCONH, -NHCSNH 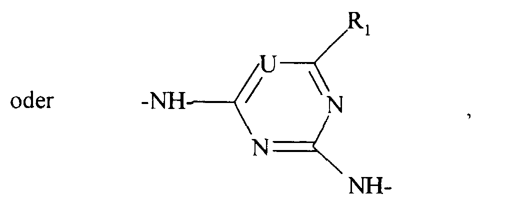
R₁ = Wasserstoff, Hydroxy, Chlor oder gegebenenfalls durch Phenyl substituiertes Amino, wobei der Phenylring durch eine Sulfonsäuregruppe substituiert sein kann,
U = N oder CH,
Spacer = eine C₂-C₆ Alkylengruppe, die gegebenenfalls durch eine Hydroxygruppe substituiert ist, eine -{(CH₂)_{g}-NH}ₙ-COCH₂-Gruppe, wobei
g = 2 - 6 und n = 0 oder 1 sein kann, oder die Gruppe 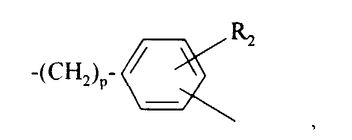
p = 0 - 4 und R₂ = Wasserstoff oder die Sulfonsäuregruppe sein kann,
Y = Valenzstrich, -NHCOCH₂, -NH-C=O, -NH-C=S, -NH-C=NH 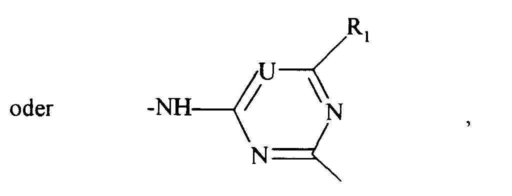 wobei R₁ und U die oben angegebenen Bedeutungen haben,
und PS = ein Polyfructosan oder Cyclodextrin darstellen wobei das Sauerstoffatom oder die Sauerstoffatome, über das/die das Polysaccharid bzw. Cyclosaccharid gebunden ist, Teil dieses Polysaccharids bzw. Cyclosaccharids ist/sind.

Bevorzugt im Sinne der Erfindung sind vor allem Verbindungen, in denen X = NH, -CONH, -SO₂NH, -NH-C=O, -NH-C=S 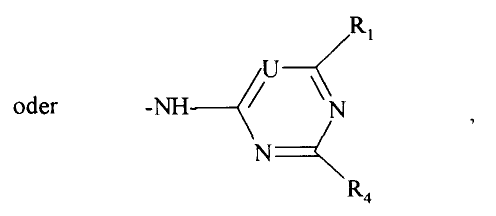
pacer = eine C₂ - C₄ Alkylengruppe, die -(CH₂)_{g}-NH-COCH₂ Gruppe mit g = 2 - 4 oder die Gruppe 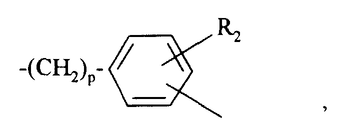 wobei p bevorzugt 0 ist und R₂ Wasserstoff oder die Sulfonsäuregruppe darstellt und R₄ Valenzstrich oder die Gruppe NH- bedeutet
Y = Valenzstrich, -NH-COCH₂, -NH-C=O 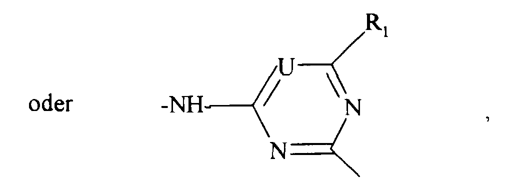
R₁ = Wasserstoff, Hydroxy oder gegebenenfalls durch Phenyl substituiertes Amino, wobei der Phenylring durch eine Sulfonsäuregruppe substituiert sein kann,
U = N und PS vorzugsweise Sinistrin, Inulin oder β-Cyclodextrin
   darstellen.

Zur besseren Wasserlöslichkeit können die Verbindungen der allgemeinen Formel I a bzw. I b gegebenenfalls nachträglich an der Polysaccharideinheit derivatisiert werden. Hierzu eignen sich insbesondere Derivate mit 2-Hydroxypropyl- bzw. Carboxymethylgruppierungen an der Polysaccharideinheit. Diese Substanzen sind ebenfalls Gegenstand dieser Erfindung.

Substanzen der allgemeinen Formel I a lassen sich nach allgemein bekannten Verfahren herstellen, vorzugsweise indem man
1. ein Farbstoffderivat der allgemeinen Formel II

   F - Z (II),

   in der
   F eine Farbstoffkomponente mit einem Absorptionsmaximum von 500 - 1300 nm,
   Z eine 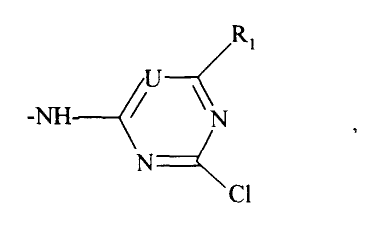
   eine -NCO, -NCS, -NC=NH, - CONCS oder die Gruppe G - Spacer - NCO, G -Spacer -NCS, G -Spacer- NCNH, 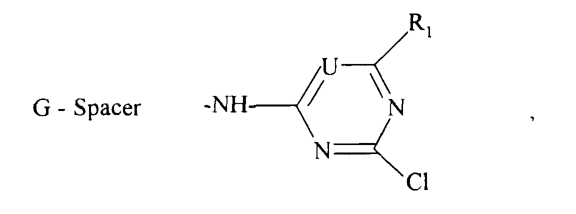 darstellt,
      wobei G = NH, -CONH, -SO₂NH, -NH-CO-NH, -NH-CS-NH bedeutet und Spacer, U und R₁ die oben angegebenen Bedeutungen haben,
      mit einem Polyfructosan oder Cyclodextrin der allgemeinen Formel III

      HO - PS (III),

      in der PS-OH eines der oben aufgeführten Polyfructosane oder Cyclodextrine darstellt,
      umsetzt
      oder
2. ein Farbstoffderivat der allgemeinen Formel IV

   F-D (IV),

   in der D eine NH₂ oder eine -G-Spacer-NH₂-Gruppe darstellt, wobei F, G und Spacer die oben angegebenen Bedeutungen haben, mit einem Polyfructosan - oder Cyclodextrinderivat der allgemeinen Formel V 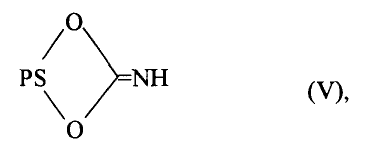 in der PS die oben angegebene Bedeutung hat,
   umsetzt
   oder
3. ein Farbstoffderivat der allgemeinen Formel IV

   F-D (IV),

   in der F und D die oben angegebenen Bedeutungen haben, mit einem Polyfructosan- oder Cyclodextrinderivat der allgemeinen Formel VI

   PS-O-CH₂-COOH (VI),

   in der PS die oben angegebene Bedeutung hat, umsetzt.
   Substanzen der allgemeinen Formel I b lassen sich nach allgemein bekannten Methoden herstellen, vorzugsweise indem man
4. ein Farbstoffderivat der allgemeinen Formel VII

   F-X-Spacer-E (VII),

   in der F, X und Spacer die oben angegebenen Bedeutungen haben und E die CHO- oder CH(OR₃)-Gruppe darstellen, wobei R₃ Methyl oder Ethyl bedeutet, mit einem Polyfructosan oder Cyclodextrin der allgemeinen Formel III

   HO-PS (III),

   in der PS die oben angegebene Bedeutung hat,
   umsetzt.

Die Herstellung eines Isocyanatderivates der allgemeinen Formel II geschieht in-situ durch thermische Zersetzung des entsprechenden Carbonsäureazids wie in Chem. Pharm. Bull. 33, 1164 (1985) beschrieben. Die Umsetzung eines Farbstoffisocyanatderivates der allgemeinen Formel II mit einem Polysaccharid oder Cyclodextrin der allgemeinen Formel III geschieht z.B. analog Makromol. Chemie 121, 18 (1968).

Isothiocyanate der allgemeinen Formel II lassen sich aus Aminen der allgemeinen Formel F-NH₂, in der F die oben angegebene Bedeutung hat, analog Chem. Ber. 63, 888 (1930), J. Chem. Soc. 125, 1702 (1924) oder Chem. Ber. 86, 314 (1953) herstellen. Deren Umsetzung mit Polysacchariden bzw. Cyclodextrinen der allgemeinen Formel III läßt sich analog Chem. Zentralblatt 1910, 910 oder Am.Chem. J. 22, 464 ***(Jahr)*** bzw. J. Am. Chem. Soc. 65, 900 (1943) durchführen.

Triazin- und Pyrimidinderivate der allgemeinen Formel II sind teilweise bekannt. So gehören z.B. die Farbstoffe Cibacron Blau und Cibacron Türkisblau GF-P zu dieser Strukturklasse. Die Herstellung geschieht im allgemeinen dadurch, daß ein Amin der allgemeinen Formel F-D, wobei F die angegebene Bedeutung hat und D eine -NH₂ oder - G-Spacer-NH₂- Gruppe darstellt, und Spacer und G die oben angegebenen Bedeutungen haben,
mit einem Triazin- oder Pyrimidinderivat der allgemeinen Formel 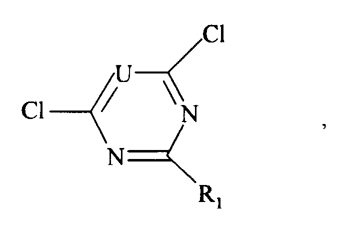 in der R₁ und U die oben angegebenen Bedeutungen haben,
umsetzt.

Diese Umsetzungen können analog J. Am. Chem. Soc. 67, 662 (1945) oder J. Am. Pharm. Assoc. 41, 385 (1952) durchgeführt werden.

Die Reaktion von Triazin- oder Pyrimidinderivaten der allgemeinen Formel II mit Polysacchariden bzw. Cyclodextrinen der allgemeinen Formel III läßt sich analog Chem. Abstr. 70, 12762 h (1969) bzw. Nature 216, 514 (1967) durchführen. Solche Umsetzungen mit Polyglucosanen sind u.a. auch beschrieben in Anal. Biochem. 31, 412 (1969) und Acta Chem. Scand. 25, 298 (1971). Hierbei wird die Reaktion in wässrigem Milieu bei einem p_{H}-Wert von 9 - 11 durchgeführt. Man kann die Reaktion jedoch auch sehr gut in aprotischen Lösungsmitteln wie N,N'-Dimethylformamid oder Toluol durchführen, wobei zur Deprotonierung Hydride wie z.B. Natriumhydrid eingesetzt werden können. Die Umsetzungen laufen dann bei Temperaturen zwischen 30 und 80°, vorzugsweise im Bereich zwischen 40 und 60° ab.

Amine der allgemeinen Formel IV, in der D eine -G-Spacer-NH₂-Gruppe darstellt, sind literaturbekannt oder werden nach an sich bekannten Methoden dargestellt. So kann man z.B. im Falle von G = CONH bzw. SO₂NH eine Carbonsäure bzw. Sulfonsäure der allgemeinen Formel F-CO₂H bzw. F-SO₃H, in der F die oben angegebene Bedeutung hat, über das entsprechende Säurechlorid mit einem Diamin der allgemeinen Formel H₂N-Spacer-NH₂, in der der Spacer die oben angegebene Bedeutung hat, unter geeigneten Reaktionsbedingungen zu einem Amid der allgemeinen Formel IV umsetzen (s. hierzu J. Med. Chem. 27, 1481 (1984), J. Prakt. Chem. 130, 293 (1931), J.Med. Chem. 34, 73 (1991) und Liebigs Ann. Chem. 1988 ,787).

Polysaccharid- oder Cyclodextrinderivate der allgemeinen Formel V werden durch Umsetzung des Polysaccharids bzw. Cyclodextrins mit Bromcyan hergestellt. Solche Verbindungen sind z.B. in Nature 214, 1302 (1967) oder Eur.J.Biochem. 18, 351 (1971) beschrieben. Die weitere Umsetzung mit Aminderivaten der allgemeinen Formel IV zu Verbindungen der allgemeinen Formel I a ist u.a. in Carbohydrate Res. 20, 1 (1971) beschrieben.

Polyfructosanessigsäuren der allgemeinen Formel VI sind u.a. in Methods Carbohydr. Chem. 6, 384 (1972) und Khim. Prir. Soedin 1969, 525 beschrieben. Cyclodextrinessigsäuren der allgemeinen Formel VI lassen sich analog darstellen.

Amine der allgemeinen Formel IV mit D = -G-(CH₂)_{g}-NH-CO-CH₂-NH₂, lassen sich durch Umsetzung von Aminen der allgemeinen Formel IV mit D = G-(CH₂)_{g}-NH₂ und Glycin darstellen, wobei die Aminosäure in Form ihres N-geschützten Aktivesters eingesetzt wird (Reaktionsbedingungen s. S. 13). Die Folgeumsetzung mit Polyfructosan- oder Cyclodextrinessigsäuren der allgemeinen Formel VI erfolgt dann analog Khim. Prir. Soedin 1969, 525.

Polyfructosan- bzw. Cyclodextrincarbonsaurederivate der allgemeinen Formel VI werden in aktivierter Form mit Aminen der allgemeinen Formel IV zur Reaktion gebracht. Als aktive Form eignen sich in diesem Fall vor allem Aktivester. So wird eine Carbonsäure der allgemeinen Formel VI mit Alkoholen wie N-Hydroxy-succinimid oder N-Hydroxy-benzotriazol in Gegenwart eines wasserabspaltenden Agenzes wie Dicyclohexylcarbodiimid in inerten Lösungsmittel wie z.B. Essigsäureethylester, Dichlormethan oder Tetrahydrofuran zur Reaktion gebracht. Der so hergestellte Aktivester wird in der Regel direkt in die weitere Reaktion unter Zusatz von organischen Basen wie Triethylamin eingesetzt.

Aldehyde bzw. Acetale der allgemeinen Formel VII lassen sich je nach der Bedeutung für X und Spacer nach an sich bekannten Methoden herstellen. So kann man z.B. eine Carbonsäure der allgemeinen Formel F-CO₂H, in der F die oben angegebenen Bedeutung hat, in Form ihres Aktivesters mit einem Aminoalkyldimethylacetal zur entsprechenden Verbindung der allgemeinen Formel VII zur Reaktion bringen (Reaktionsbedingungen s. S. 13).

Die Reaktion eines Aldehyds bzw. Acetals der allgemeinen Formel VII mit einem Polyfructosan oder Cyclodextrin der allgemeinen Formel III wird vorzugsweise in dipolar aprotischen Lösungsmitteln wie Essigsäurethylester, N-Methylpyrrolidon oder N,N 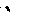 -Dimethylpropylenharnstoff bei Temperaturen zwischen 0°C und 30°C in Gegenwart von katalytischen Mengen Säure wie z.B. p-Toluolsulfonsäure, Bortrifluorid oder Zinkchlorid durchgeführt.

Die nachträgliche Überführung von Verbindungen der allgemeinen Formel I a bzw. I b in wasserlöslichere Substanzen durch Umsetzung mit Chloressigsäure bzw. Propenoxyd läßt sich analog literaturbekannter Verfahren durchführen. So ist die Umsetzung eines Polysaccharids mit Chloressigsäure u.a. in Methods Carbohydr. Chem. 6, 384 (1972) oder Makromol. Chem. 122, 271 (1969) und die Reaktion eines Polysaccharids mit Propenoxyd in 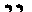 Starch Chemistry and Technology", Academic Press New York (1984), R.L. Whistler et al. beschrieben.

Die erfindungsgemäßen Verbindungen der allgemeinen Formel Ia oder Ib werden in der Regel intravenös verabreicht. Hierzu werden 10 - 25% ige isotonische, wässrige Lösungen eingesetzt, die die bei Injektionen üblichen Zusätze wie Stabilisierungsmittel und Puffer enthalten. Als Puffer werden vorzugsweise Natrium- oder Kaliumphosphatpuffer verwendet, als Stabilisierungsmittel können gegebenenfalls Antioxidantien zum Einsatz kommen. Um eine isotone Lösung zu erhalten, wird Natriumchlorid oder Mannit zugesetzt. Der pH-Wert der Lösung liegt zwischen 6,5 und 8, vorzugsweise zwischen pH = 7 und 7,5. Die erfindungsgemäßen Verbindungen der allgemeinen Formel Ia oder Ib werden einmalig in einer Menge von 0,1 - 5 g, vorzugsweise 0,2 - 2 g appliziert.

### Beispiel 1

### Reaktionsprodukt von 1-Amino-4-{4-[4-chlor-6-(4-hydroxysulfonyl-phenylamino)-〈1.3.5〉triazin-2-ylaminol] 3-hydroxysulfonyl-phenylamino} 9.10-dioxo-9.10-dihydroanthracen-2-sulfonsäure (= Cibacron Blue F3GA) mit Dahlien-Inulin

1g Dahlien-Inulin ( Fluka ) werden in 10 ml destilliertem Wasser suspendiert und auf 50°C erhitzt. Man tropft 0,1 g Cibacron Blue F3GA ( Aldrich ) in 10 ml destilliertem Wasser zu. Anschließend gibt man eine Lösung von 2 g Natriumsulfat und 0,232 g Natriumphosphat-dodecahydrat in 3 ml destilliertem Wasser zu. Man hält die Lösung 3 Stunden bei 50°C, kühlt ab und neutralisiert durch Zugabe von 1 N Salzsäure. Anschließend wird der Ansatz durch Dialyse (Dialyseschlauch Spectra/Por 6 der Firma Roth/Karlsruhe) gegen destilliertes Wasser entsalzt und von nicht umgesetztem Farbstoff befreit. Nach Beendigung der Dialyse wird die Lösung eingeengt und getrocknet. Man erhält 0,45 g der gewünschten Substanz. λ max: 610 nm (Wasser), 629 nm ( Dimethylsulfoxyd)

### Beispiel 2

### Reaktionsprodukt von 1-Amino-4-{4-[4-chlor-6-(4-hydroxysulfonylphenylamino)-〈1.3.5〉triazin-2-ylamino]3-hydroxysulfonyl-phenylamino}9.10-dioxo-9.10-dihydroanthracen-2-sulfonsäure(=Cibacron Blue F3GA) mit β-Cyclodextrin

Zu 1 g β-Cyclodextrin (0,88 mmol) in 10 ml Wasser gibt man 0,4 g 50-prozentige Natronlauge zu. Man läßt 1 Stunde unter Stickstoff rühren, erhitzt auf 70°C und gibt im Verlauf von 4 Stunden portionsweise jeweils 200 mg Cibacron F3GA (Aldrich) in 36 ml Wasser zu. Nach weiteren 30 Minuten läßt man die Lösung abkühlen, neutralisiert mit 1 N Salzsäure und dialysiert den Ansatz gegen destilliertes Wasser (Dialyseschlauch Spectra/Por MWCO:2.000). Hierbei wird die Lösung entsalzt und von nicht umgesetztem β-Cyclodextrin und Farbstoff befreit. Anschließend wird die Lösung eingeengt und getrocknet. Man erhält 430 mg eines blauen Feststoffes; r_{F}-Wert: 0,78 (RP-18, Methanol/Wasser = 1/1).

### Beispiel 3

Weitere bevorzugte Verbindungen im Sinne der Erfidnung sind die folgenden:

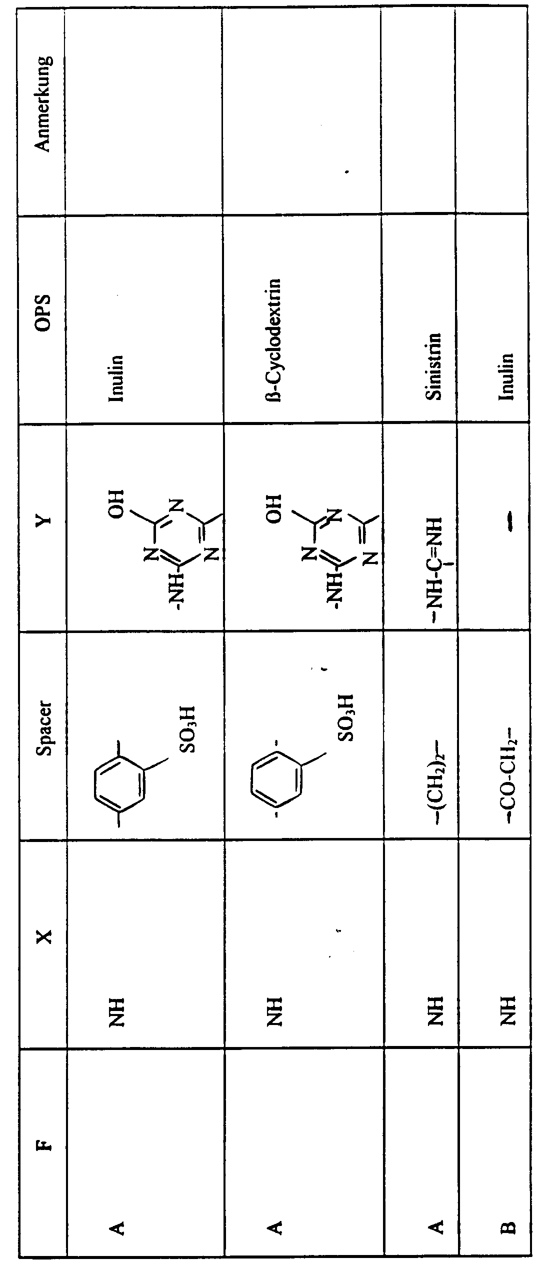

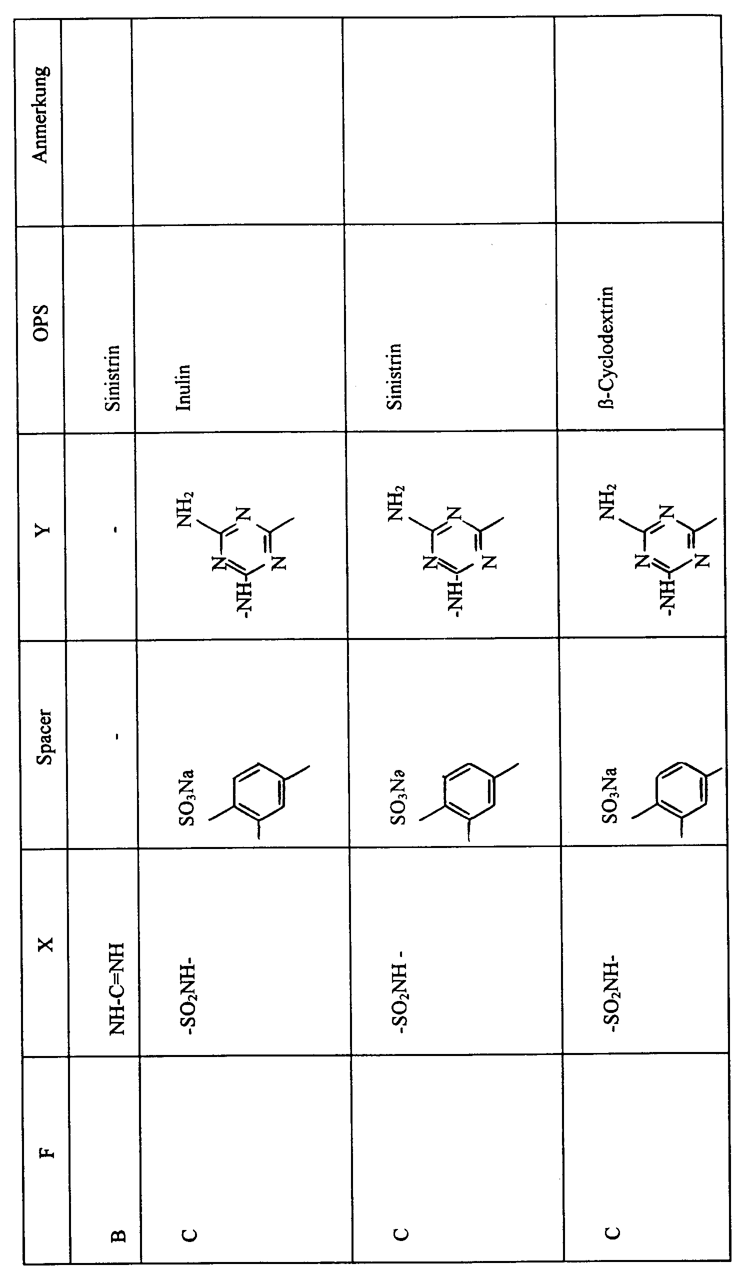

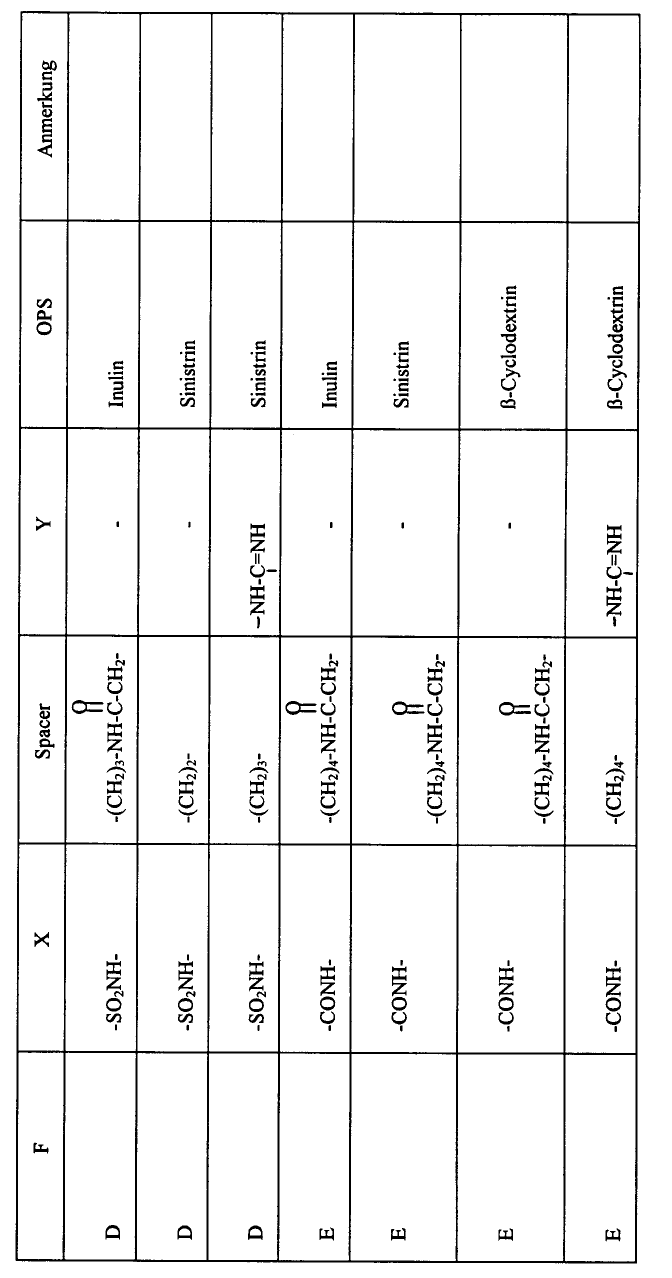

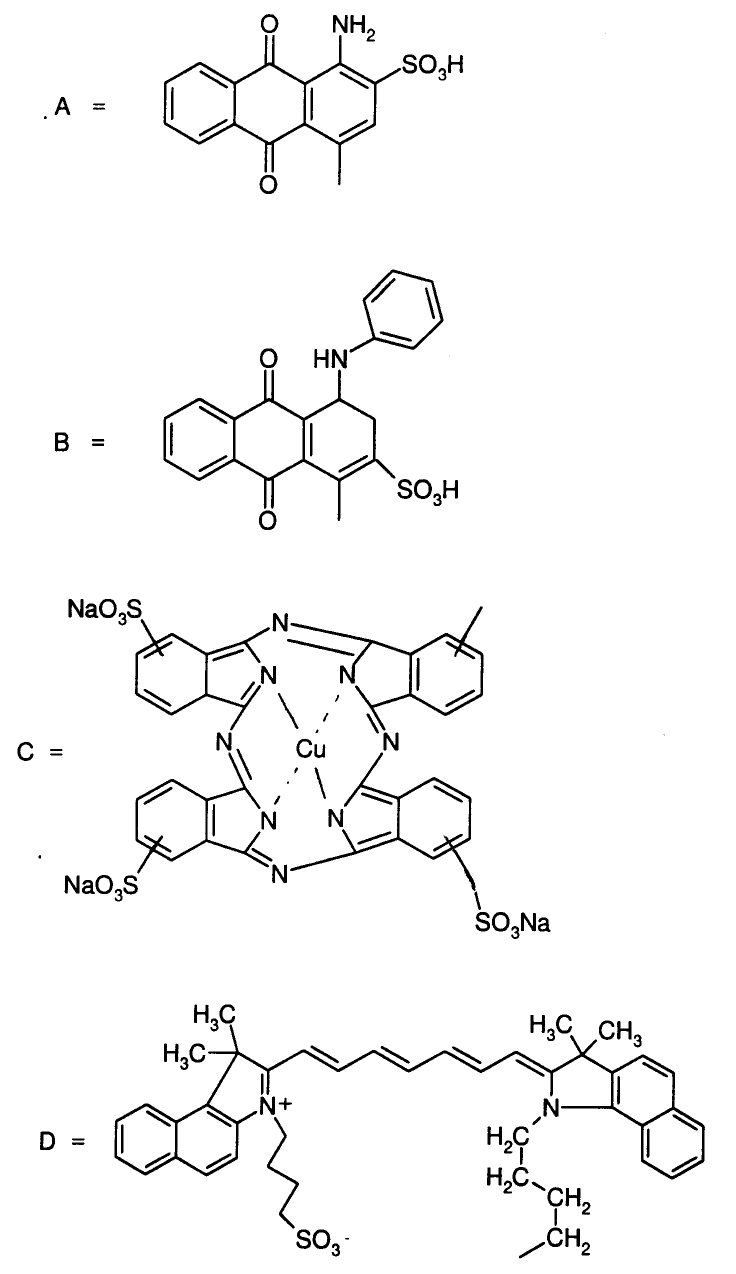

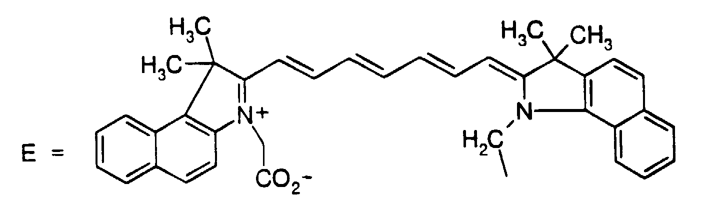

## Patentansprüche

1. Verbindungen der Formel 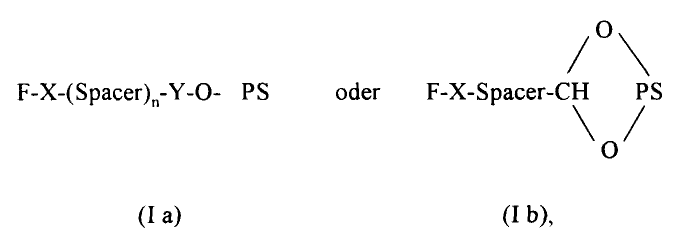 in der
F = eine Farbstoffkomponente mit einem Absorptionsmaximum zwischen 500 und 1300 nm, vorzugsweise zwischen 600 und 900 nm,
n = 0 oder 1,
X für den Fall, daß n = 0 ist, =
-NH-C=O, -NH-C=S, -NH-C=NH, -CONH-C=S 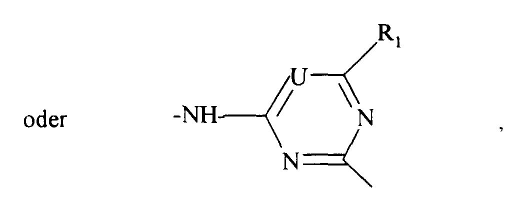
X für den Fall, daß n = 1 ist, =
NH, -CONH, -SO₂NH, -NHCONH, -NHCSNH 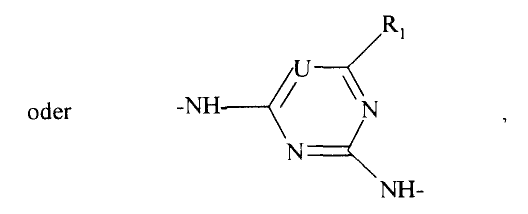
R₁ = Wasserstoff Hydroxy, Chlor oder gegebenenfalls durch Phenyl substituiertes Amino, wobei der Phenylring durch eine Sulfonsäuregruppe substituiert sein kann,
U = N oder CH,
Spacer = eine C₂-C₆ Alkylengruppe, die gegebenenfalls durch eine Hydroxygruppe substituiert ist, eine -{(CH₂)_{g}-NH}ₙ-COCH₂-Gruppe, wobei
g = 2 - 6 und n = 0 oder 1 sein kann, oder die Gruppe 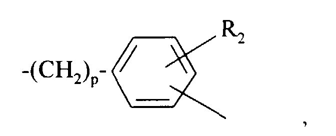
p = 0 - 4 und R₂ = Wasserstoff oder die Sulfonsäuregruppe sein kann,
Y = Valenzstrich, -NHCOCH₂, -NH-C=O, -NH-C=S, -NH-C=NH 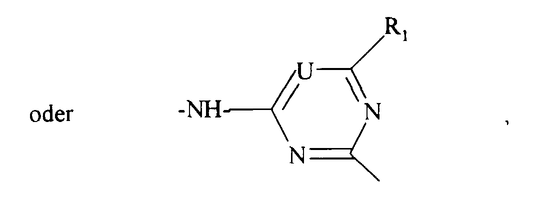 wobei R₁ und U die oben angegebenen Bedeutungen haben,
und PS = ein Polyfructosan oder Cyclodextrin darstellen, wobei das Sauerstoffatom oder die Sauerstoffatome, über das/die das Polysaccharid bzw. Cyclosaccharid gebunden ist, Teil dieses Polysaccharids bzw. Cyclosaccharids ist/sind.

2. Verbindungen der Formel I(a) oder I(b) gemäß Anspruch 1, in denen X = NH, -CONH-, -SO₂NH, -NH-C=O, -NH-C=S 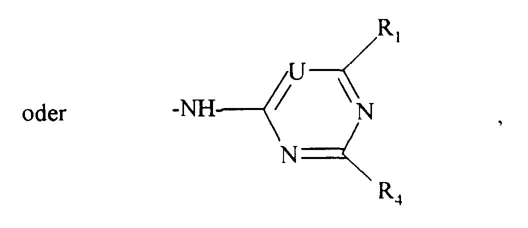
Spacer = eine C₂ - C₄ Alkylengruppe, die -(CH₂)_{g}-NH-COCH₂ Gruppe mit g = 2 - 4 oder die Gruppe 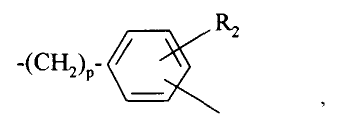 wobei p bevorzugt 0 ist und R₂ Wasserstoff oder die Sulfonsäuregruppe darstellt und R₄ Valenzstrich oder die Gruppe NH- bedeutet.
Y = Valenzstrich, -NH-COCH₂, -NH-C=O 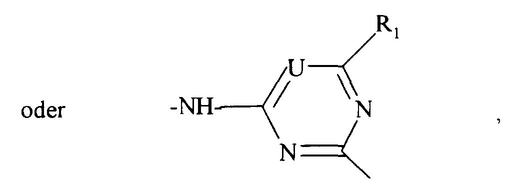
R₁ = Wasserstoff , Hydroxy oder gegebenenfalls durch Phenyl substituiertes Amino, wobei der Phenylring durch eine Sulfonsäuregruppe substituiert sein kann,
U = N
und PS vorzugsweise Sinistrin, Inulin oder β-Cyclodextrin darstellen.

3. Diagnostisches Mittel, enthaltend eine Verbindung gemäß Anspruch 1 oder 2.

4. Diagnostisches Mittel gemäß Anspruch 3, dadurch gekennnzeichnet, daß es zur Bestimmung der glomerulären Filtrationsrate beim Menschen eingesetzt wird.

5. Verwendung von Verbindungen gemäß Anspruch 1 oder 2 als Diagnostikum zur Bestimmung der glomerulären Filtrationsrate beim Menschen.

6. Verfahren zur Bestimmung der glomerulären Filtrationsrate unter Verwendung eines Farbstoffes gemäß Anspruch 1 oder 2, dadurch gekennzeichnet, daß man in zeitlichen Abständen oder kontinuierlich die Konzentration des Farbstoffes bestimmt.

7. Verfahren gemäß Anspruch 6, dadurch gekennzeichnet, daß die Bestimmung nicht invasiv erfolgt.
